# EUROPEAN PATENT SPECIFICATION

(11) **EP 3 935 191 B1**
(45) Date of publication and mention of the grant of the patent: **01.04.2026**
(21) Application number: 19835860.8
(22) Date of filing: 02.01.2019
(51) Int. Cl.: C12Q 1/6886

(54) **STRESS-REGULATED MIRNAS AS NOVEL CANCER BIOMARKERS**
STRESSREGULIERTE MIRNAS ALS NEUARTIGE KREBSBIOMARKER
MICRO-ARN À CONTRAINTE RÉGULÉE UTILISÉS EN TANT QUE NOUVEAUX BIOMARQUEURS DU CANCER

(43) Date of publication of application: 12.01.2022
(73) Proprietor: Sabanci Üniversitesi Nanoteknoloji Arastirma Ve Uygulama Merkezi Sunum, 34956 Istanbul (TR); Sabanci Üniversitesi, 34956 Tuzla/Istanbul (TR)
(72) Inventor: GOZUACIK, Devrim, 34956 Istanbul (TR); OZTURK, Deniz Gulfem, 34956 Istanbul (TR); KOCAK, Muhammed, 34956 Istanbul (TR)
(74) Representative: Mutlu, Aydin
(86) International application number: PCT/TR2019/050004
(87) International publication number: WO 2020/142017

(56) References cited:
- CA-A1- 3 018 048
- US-A1- 2017 130 275
- US-A1- 2017 166 975
- DI LEVA GIANPIERO ET AL: "miRNA profiling of cancer", CURRENT OPINION IN GENETICS & DEVELOPMENT., vol. 23, no. 1, 1 February 2013 (2013-02-01), XX, pages 3 - 11, XP093151254, ISSN: 0959-437X, DOI: 10.1016/j.gde.2013.01.004
- J. DRAHOS ET AL: "MicroRNA Profiles of Barrett's Esophagus and Esophageal Adenocarcinoma: Differences in Glandular Non-native Epithelium", CANCER EPIDEMIOLOGY, BIOMARKERS AND PREVENTION., vol. 25, no. 3, 24 November 2015 (2015-11-24), US, pages 429 - 437, XP055676691, ISSN: 1055-9965, DOI: 10.1158/1055-9965.EPI-15-0161
- L V GAYOSSO-GÓMEZ ET AL: "IDENTIFICATION OF CIRCULATING MiRNA PROFILES THAT DISTINGUISH MALIGNANT PLEURAL MESOTHELIOMA FROM LUNG ADENOCARCINOMA", EXCLI JOURNAL, 1 January 2014 (2014-01-01), pages 740 - 750, XP055613982, Retrieved from the Internet <URL:https://www.ncbi.nlm.nih.gov/pmc/articles/PMC4464514/pdf/EXCLI-13-740.pdf> [retrieved on 20190820]
- ABDULLAH MORIDIKIA ET AL: "MicroRNAs: Potential candidates for diagnosis and treatment of colorectal cancer", JOURNAL OF CELLULAR PHYSIOLOGY, vol. 233, no. 2, 15 May 2017 (2017-05-15), US, pages 901 - 913, XP055676640, ISSN: 0021-9541, DOI: 10.1002/jcp.25801

## Description

### Technical Field

The present invention relates to nucleic acid(s) and combinations thereof for the diagnosis and/or follow-up of cancer, which are used for the determination of expression of microRNA molecules (hereinafter, referred to as "miRNA"s). More specifically, the invention relates to a method involving nucleic acid(s) and combinations thereof capable of specifically binding to certain miRNA molecule(s), which can be used as novel non-invasive cancer biomarkers for the early diagnosis and follow-up of cancer, especially of colorectal cancer, liver cancer (hepatocellular carcinoma), pancreas cancer, and stomach cancer. Furthermore, the invention pertains to a method for diagnosis of cancer making use of said nucleic acid(s) and combinations thereof which are complementary to the miRNAs of the present invention, which can be utilized as cancer biomarkers.

### Background of the Invention

Macroautophagy (autophagy herein) is a highly conserved cellular stress response and a survival pathway that is responsible for the degradation and recycling of long-lived proteins, protein aggregates, intracellular pathogens, as well as damaged organelles in order to maintain cellular homeostasis. Autophagy is rapidly upregulated under stress conditions. Among various stress factors, the most typical reason triggering autophagy is the nutrient starvation. When exposed to starvation, the cell or the organism increases its autophagic activity in order to adapt to the limiting conditions. Unnecessary proteins are degraded and acquired amino acids are recycled for the synthesis of proteins that are essential for survival.

Autophagy is associated with the most of the basic cellular mechanisms. Being a key guardian of many cellular pathways, abnormalities of autophagy lead to health problems almost invariably. Said health problems include neurodegenerative diseases and cancers. According to the current view, autophagy is thought to serve as a tumor suppressor in the early phases of cancer formation. Yet in later phases, autophagy may support and/or facilitate tumor growth, spread and contribute to treatment resistance. Therefore, autophagy is considered as a stage-dependent dual player in cancer.

Cancer can be defined as group of diseases that are characterized with abnormal cell growth and have the potential to spread to other parts of the body through the blood and lymph systems. There are over 100 types of cancers that affect humans, which are classified according to the type of cells they initially affect

Detection and diagnosis of cancer can be achieved by the examination of the symptoms or through screening. Cancer screening's purpose is to detect the cancer before symptoms arise, through tests and medical imaging. In addition to screening, early diagnosis plays an important role on the survival rate of cancer patients. As the understanding of cancer biology improves and medical technology advances, more cases are detected accurately in earlier stages and the success of the treatment increases.

In addition to being effective and accurate, advancements in cancer detection technologies are also preferred to be non-invasive. Developing minimally invasive molecular markers that can detect the presence of cancer cells in the blood or in any other body fluid of a patient in the early stages of cancer is of great interest in diagnostic cancer studies.

Tumor markers are biomarkers found in biological fluids and/or body tissues, that can be detected in the presence of cancer through the alteration in their levels. They can be produced by the tumor cells themselves, or by other cells as a response to the tumor. An ideal tumor marker preferably possesses the following properties: high specificity (not detected in benign diseases and control subjects), high sensitivity (detected in early stages of cancer), and correlation with the stage of the case. Especially blood-based tumor markers are gaining acceptance as a potential alternative for non-invasive detection of cancer.

There are many tumor markers which are currently in use. These classical cancer biomarkers include carcinoembryonic antigen (CEA) and carbohydrate antigen 19-9 (CA19-9). CEA can be defined as a set of glycoproteins which are related to cell adhesion. For healthy adults, the levels of CEA in the blood is quite low. However, CEA levels may rise in several types of carcinoma, as well as some non-neoplastic conditions. The cancer types for which elevated CEA levels are detected include colorectal carcinoma, gastric carcinoma, pancreatic carcinoma, lung carcinoma, and breast carcinoma. CA19-9 on the other hand, is an antigen defined by its corresponding monoclonal antibody. It is used particularly for the detection of pancreatic cancer. Likewise, its levels may also rise in colorectal cancer.

Colorectal cancer (CRC) is the development of cancer from the epithelial cells that are lining the colon or rectum of the gastrointestinal tract It is one of the most common malignancies worldwide, being the second most common cause of cancer in women and the third most common in men. Overall, it is the fourth most common cause of cancer death after lung, stomach, and liver cancers. Survival is related to the stage of detection and the type of cancer involved. Survival rate is low for symptomatic cancers. Since the indication of colorectal cancer in the initial phase is not clearly observable, symptomatic cases are generally quite advanced. On the other hand, survival rates for early stage detection are about five times greater than that of late stage cancers. Diagnosis typically involves sampling of areas of the colon that are suspicious for possible tumor development through colonoscopy. Blood-based tests can also be used for detection of the disease, with the target molecules being CEA and CA19-9. WO2016/156128 A1 document relates to a method for the diagnosis and/or track of prognosis of cancer in patients (wherein the said cancer can be selected from many types of cancer, including colorectal cancer), by making use of biological markers including CEA.

Liver cancer is the type of cancer that primarily originates in the liver. Hepatocellular carcinoma (HCC) is the most common primary liver malignancy throughout the world and is the third leading cause of cancer-related death. Unlike other cancer types, HCC generally emerges on a previously damaged organ. The most important risk factor for the development of HCC is cirrhosis. Moreover, chronic infections of hepatitis B and/or hepatitis C can attribute to the progression of hepatocellular carcinoma, since these infections cause the body's own immune system to attack the liver cells repeatedly. Diagnosis involves blood-testing and imaging for both asymptomatic patients and those with symptoms of liver disease. AFP (α-fetoprotein) is currently the most widely used biomarker for HCC (Attwa MH, El-Etreby SA. "Guide for diagnosis and treatment of hepatocellular carcinoma" World J Hepatol (2015) 7(12):1632-51).

Pancreas is a glandular organ behind the stomach. Pancreatic cancer (also referred as pancreas cancer) arises when cells in the pancreas begin to multiply uncontrollably. Other organs may also be invaded by these cancerous cells. Most cases of pancreatic cancer starts within the part of the pancreas where digestive enzymes are produced. Pancreatic cancer typically does not cause noticeable symptoms in its early stages, therefore the condition is not diagnosed until it spreads to the other parts of the body. Diagnosis of pancreatic cancer involves imaging and blood tests. As a tumor marker, CA19-9 is commonly used in the diagnosis of pancreatic cancer. WO2017/008389 A1 document claims a kit for detecting pancreatic cancer protein biomarkers, wherein CA19-9 is included within the said biomarkers.

Stomach cancer (also known as gastric cancer) develops from the lining of the stomach. It is the fifth leading cause of cancer globally and ranks number three in cause of death from cancer. Infection by the bacterium Helicobacter pylori is the most common cause of stomach cancer, accounting for more than 60% of cases. If detected early, many cases can be cured. But the diagnosis mostly takes place in the late stages, since stomach cancer usually doesn't cause any early symptoms. Diagnosis usually makes use of biopsy, which is performed during endoscopy and followed by imaging tests in order to determine if there is metastasis. As for biomarkers, CEA and CA19-9 are the standard tumor markers for the stomach cancer (Jin Z, Jiang W, Wang L. "Biomarkers for gastric cancer: Progression in early diagnosis and prognosis (Review)" Oncol Lett 2015;9(4):1502-1508).

miRNAs (micro RNAs) are a class of single stranded non-coding RNAs. They are usually comprised of 19-25 nucleotides. They play a key role in the regulation of post-transcriptional gene expression. Interaction between a miRNA and any given of its mRNA targets results in degradation or translational repression of said gene transcripts. miRNAs are abundant in many mammalian cell types and they regulate 60% of the genes of humans.

The activity of miRNAs are associated with the control of a wide range of cellular mechanisms such as cell growth, differentiation, cell proliferation, apoptosis and migration pathways. Analysis of differential expression of miRNAs constitutes the basis for the understanding of miRNA function. Moreover, since their functioning is essential for basic mechanisms of cell, the dysregulation of miRNA levels contribute to development and prognosis of various diseases, including many types of cancers. Accumulating data in the literature indicate that dysregulation of miRNA expression contribute to mechanisms of cancer formation, invasion, metastasis, and affect responses to chemotherapy or radiotherapy. Certain microRNAs can display different concentration or expression levels in tissue and blood depending on cancer type and stage, and therefore may be suitable biomarkers for early detection of different cancers. In fact, the first human disease to be correlated with the deregulation of miRNAs is chronic lymphocytic leukemia (Musilova K, Mraz M (May 2015). "MicroRNAs in B-cell lymphomas: how a complex biology gets more complex". Leukemia. 29 (5): 1004-17).

Therefore, valuable diagnostic and prognostic indications concerning human malignancies can be deduced from specific miRNA expression patterns. Changes in specific miRNA levels were reported in almost all types of malignancies, including lung cancer, colon cancer, pancreatic cancer, and breast cancer (Võsa U, Vooder T, Kolde R, Fischer K, Välk K, Tõnisson N, Roosipuu R, Vilo J, Metspalu A, Annilo T (October 2011). *"Identification of miR-374a as a prognostic marker for survival in patients with early-stage nonsmall cell lung cancer".* Genes, Chromosomes & Cancer. 50 (10): 812-22; Eyking A, Reis H, Frank M, Gerken G, Schmid KW, Cario E (2016). "MiR-205 and MiR-373 are associated with aggressive human mucinous colorectal cancer" PLOS One. 11 (6): e0156871; Xu YF, Hannafon BN, Zhao YD, Postier RG, Ding WQ (September 2017). "Plasma exosome miR-196a and miR-1246 are potential indicators of localized pancreatic cancer". Oncotarget 8 (44): 77028-77040; Wu H, Mo YY (December 2009). "Targeting miR-205 in breast cancer". Expert Opinion on Therapeutic Targets. 13 (12): 1439-48).

Differential expression of miRNAs between tumors and their corresponding normal tissues led them to be introduced as potent cancer markers. The usage of miRNAs as cancer biomarkers enables a diagnosis with high specificity and high sensitivity. Moreover, miRNAs ensure a non-invasive way of diagnosis. US2017/073764 A1 document relates to a method assisting the detection of pancreatic cancer with high accuracy. Said method makes use of certain miRNA molecules (miR-122-5p, miR-16-5p, miR-19b-3p and miR-25-3p) as indicators for the presence of cancer.

The pioneering discovery that miRNAs also circulate in cell-free blood plasma and serum was later confirmed by the the presence of circulating miRNAs in all other biological fluids. These miRNAs circulate in the bloodstream in a highly stable extracellular forms, are overexpressed in cancer and are detectable within the diagnostic laboratory. Accumulated studies have shown that circulating miRNAs can be protected from degradation through formation of AGO-miRNA complex or encapsulation into exosomes, microvesicles, and HDL particles. The expression profile of these miRNAs shows great promise as a novel non-invasive biomarker for diagnosis of cancer. WO2017/144642 A1 document claims compositions and methods for analyzing and characterizing patients suffering from colon cancer by the detection of specific circulating miRNAs. The aim of the application is to predict the clinical development of colorectal cancer. A review titled *"MicroRNAs: Potential candidates for diagnosis and treatment of colorectal cancer"* discloses the findings of preclinical and clinical studies performed on tissue-specific and circulating miRNAs as diagnostic biomarkers and therapeutic targets for the detection of patients at various stages of CRC (Abdullah Moridikia et al. "MicroRNAs: Potential candidates for diagnosis and treatment of colorectal cancer", Journal of Cellular Physiology, vol. 233, no. 2, 15 May 2017 (2017-05-15), pages 901-913).

The technical field about the relationship between the certain miRNAs and cancer is to be improved. The determination of the certain miRNAs that are dysregulated in each type of cancer may lead to a promising development in non-invasive diagnostics of cancer. In consideration of these points, new studies and discoveries are essential towards the efficient and accurate early detection of cancer by the utilisation of miRNAs.

### Brief Description of the Figures

Figure 1 shows differentially expressed microRNAs under starvation condition.
Figure 2 shows the qRT-PCR validation on 21 upregulated miRNA molecules' responses in MCF-7 cells with or without starvation treatment.
Figure 3 shows the qRT-PCR expression analysis for miR-4488. The graphic compares the miR-4488 levels of colon cancer tissue samples from patients to non-tumor colon tissue samples from the same patients.
Figure 4 shows the ratio of tumors with high (black) or low (white) miR-4488 levels relative to the non-tumor samples of the same patients. (Fold change threshold=1,5)
Figure 5 shows the qRT-PCR expression analysis for miR-4492. The graphic compares the miR-4492 levels of colon cancer tissue samples from patients to non-tumor colon tissue samples from the same patients.
Figure 6 shows the ratio of tumors with high (black) or low (white) miR-4492 levels relative to the non-tumor samples of the same patients. (Fold change threshold=1,5)
Figure 7 shows the qRT-PCR expression analysis for miR-4488 or miR-4492 in colon cancer samples from patients compared to non-tumor colon tissue samples from the same patients.
Figure 8 shows the ratio of tumors with high (black) or low (white) miR-4488 or miR-4492 levels relative to the non-tumor samples of the same patients. (Fold change threshold=1,5)
Figure 9 shows the qRT-PCR analysis for miR-4488 expression in blood samples from 5 healthy volunteers and 11 colon cancer patients. (CNT = control group, CRC = colorectal cancer patients)
Figure 10 shows the ratio of blood samples with high (black) or low (white) miR-4488 levels relative to the average of control samples.
Figure 11 shows the qRT-PCR analysis for miR-4492 expression in blood samples from 5 healthy volunteers and 11 colon cancer patients. (CNT = control group, CRC = colorectal cancer patients)
Figure 12 shows the ratio of blood samples with high (black) or low (white) miR-4492 levels relative to the average of control samples.
Figure 13 shows the qRT-PCR results of miR-4488 and miR-4492 in tumor vs non-tumor samples for hepatocellular cancer.
Figure 14 shows the qRT-PCR results of miR-4488 and miR-4492 in tumor vs non-tumor samples for pancreas cancer.
Figure 15 shows the qRT-PCR results of miR-4488 and miR-4492 in tumor vs non-tumor samples for stomach cancer.

### Detailed Description of the Invention

In this detailed description, the method of diagnosis and follow-up of cancer by the analysis of miRNA molecules are described for better understanding of the subject matter.

Autophagy can be defined as an intracellular degradation system during which long-lived proteins, protein aggregates, and damaged organelles are eliminated through lysosomal degradation. The pathway is activated upon stress conditions, such as nutrient starvation. Being at the crossroads of various cellular response pathways and mechanisms, dysregulation of autophagy might result in pathological states including cancer.

Colorectal cancer (CRC) is defined as abnormal cell growth originating from epithelial cells of colon or rectum. CRC is one of the most common types of cancer globally. Survival rate is in low levels, since the symptoms generally begin to arise when the disease is quite advanced. Classical diagnosis is generally performed with biopsy, which is an invasive diagnostic method. Also, serum tests that target certain antigens (CEA and CA19-9) are used. CEA is the most widely used and accepted blood-based protein biomarker for colorectal cancer. But detection of this serum antigen marker is not an effective method for CRC screening, because elevated levels of CEA are only detected in advanced stages and in a fraction of CRC patients. Moreover, CEA is not specific for CRC, and elevated levels may be observed due to other diseases such as IBD, liver disease, pancreatitis, and other malignancies. Similarly, CA19-9 is not specific and effective enough for an accurate diagnosis of CRC.

Hepatocellular carcinoma (HCC) is the deadliest liver malignancy globally. In most cases, the disease affects the liver that is already damaged due to cirrhosis or hepatitis infections. Unlike most malignancies, the diagnosis of HCC can be performed non-invasively with blood-based tests and imaging. Most commonly used biomarker for HCC is AFP. However, this protein has poor sensitivity and specificity as a tumor marker. Therefore, it is unfavorable in the terms of efficiency and accuracy. Furthermore, present tumor markers are typically useful in combination with each other, meaning their sensitivity and specificity are not reliable on their own.

Pancreatic cancer can be defined as abnormal growth of tumor cells in the pancreas. The region of pancreas that is responsible for the production of digestive enzymes is usually the starting point of the cancer. Laboratory tests, which are followed by imaging tests are typically used during the diagnosis and detection of prognosis. CA19-9 is the most commonly used tumor marker for pancreatic cancer. However, required level of sensitivity and specificity are not provided by this antigen. Moreover, the use of CA19-9 for screening of pancreas in discouraged due to high number of false negative and false positive results. Therefore, CA19-9 is favorably used for follow-up of known cases, rather than diagnosis and detection.

Tumor formation in the lining of the stomach is called stomach cancer. Most cases arise due to bacterium Helicobacter pylori infection. Early detection has vital importance, as it is for other types of cancer. Biopsy and imaging techniques are widely used for diagnosis. As regards to tumor markers, the most commonly used tumor markers in stomach cancer are CEA and CA19-9. However, as described above for other types of cancer, low sensitivity and specificity of these tumor markers make them unfavorable for the use in initial diagnosis of stomach cancer.

In recent years, use of miRNA molecules as a diagnostic tool for cancer have been introduced as an alternative for classical biomarkers. miRNAs have been found to have a promising diagnostic value for cancer. miRNAs are short non-coding RNA molecules that are responsible for the post-transcriptional regulation of gene expression. They perform this action through degradation or repression of target mRNAs. miRNAs play an important role in many basic cellular mechanisms. For that matter, aberrant miRNA levels are associated with various diseases, including cancer. Dysregulated levels of miRNAs, which are determined through differential expression studies, can be an accurate proof of a presence of a tumor. Based on this fact, new techniques and tests can be developed in order to diagnose cancer effectively. In fact, since the introduction of miRNAs as potential tumor markers, many studies which are revealing the relationship between certain miRNA molecules with certain types of cancer have been released. Along with being sensitive and specific, the use of miRNA molecules as tumor markers also enables a non-invasive diagnosis and detection method. In addition to these advantages, circulating miRNAs can also be present extracellularly in cell-free blood plasma/serum and all other biological fluids in stable levels. Therefore, more types of samples can be used for diagnosis, without the need for an invasive approach. These features make miRNA molecules efficient and favorable biomarkers in the field of cancer diagnostics.

In the light of the points mentioned above, considering the emerging role of autophagy in health and disease, identification of novel autophagy-associated miRNAs and determination of relations between miRNA expression and physiological/pathophysiological conditions might contribute a better understanding of cancer diagnosis and progression. Considering this fact, the present invention identifies new autophagy-associated miRNAs that are induced by starvation and describes a method of cancer diagnosis by the utilisation of these newly identified miRNAs as blood-based cancer biomarkers.

The inventors of the current invention have realized that the levels of miR-4488 and/or miR-4492 remarkably change in tumoral tissues. Using nucleic acid(s) and combinations thereof which are capable of binding to said miRNA molecules for the diagnosis of cancer, especially of the cancers selected from the group consisting of colorectal cancer, hepatocellular carcinoma, pancreatic cancer and stomach cancer, would improve reliability of the diagnosis while providing a practical way of detection and follow-up of the cancer in a non-invasive way.

The inventors have therefore concluded that nucleic acid(s) and combinations thereof capable of specifically binding to miR-4488 (SEQ ID NO: 1) and/or miR-4492 (SEQ ID NO: 2) (i.e. biomarkers) may be useful for use in diagnosis of cancer. Said nucleic acid(s) may involve complementary sequences of miR-4488 or miR-4492.

Preferably, nucleic acid(s) and combinations thereof capable of specifically binding to miR-4488 (SEQ ID NO: 1) and miR-4492 (SEQ ID NO: 2) may be useful for use in diagnosis of cancer.

Said cancer is preferably selected from the group consisting of colorectal cancer, liver cancer (e.g. hepatocellular carcinoma), pancreatic cancer and stomach cancer. The cancer is preferably colorectal cancer.

The present invention further relates to miR-4488 and complementary nucleic acid(s) thereof for use in diagnosis of colorectal cancer.

In an embodiment which is not covered by the subject-matter of the claims, present invention relates to a method for the diagnosis and/or follow-up of prognosis of cancer comprising the evaluation of miR-4488 and/or miR-4492 molecules as biomarkers. Said cancer is preferably selected from the group consisting of colorectal cancer, liver cancer, pancreatic cancer and stomach cancer.

In an aspect, the present invention provides a method for the diagnosis and/or follow-up of prognosis of colorectal cancer comprising the evaluation of miR-4488 molecule as a biomarker.

The differential expression analysis of miR-4488 and/or miR-4492 may be performed in a sample obtained from a patient who has or is suspected of having one of the types of the cancers listed above. Said sample can be a blood sample, a body fluid, or a tissue sample.

In an embodiment of the present invention, the control or reference sample of the present invention can be a blood sample or a body fluid from a healthy individual, or a healthy tissue sample from a patient.

It is put forward by the present inventors that the altered abundance of miR-4488 and/or miR-4492 in a sample relative to a reference or control sample indicates a higher likelihood of colorectal cancer, hepatocellular carcinoma, pancreatic cancer or stomach cancer. Accordingly, these miRNAs can be useful in a method for the diagnosis of cancer, wherein the altered abundance of miR-4488 and miR-4492 in a sample relative to a reference or control sample indicates a higher likelihood of colorectal cancer, hepatocellular carcinoma, pancreatic cancer or stomach cancer. Such method is not covered by the subject-matter of the claims.

The expression levels of miR-4488 and/or miR-4492 are considered statistically high/low relative to a reference or control sample when the fold change is higher than 1.5. Preferably, the expression levels of miR-4488 and miR-4492 are considered statistically high/low relative to a reference or control sample when the fold change is higher than 1.5.

In an embodiment which is not covered by the subject-matter of the claims, one of the nucleic acid(s) capable of specifically binding to miR-4488 or miR-4492 can be replaced with fragments or variants, or modified nucleic acid(s) with additional chemical moiety/moieties capable of specifically binding to at least one miRNA selected from the group consisting of miR-4532 (SEQ ID NO: 10), miR-3656 (SEQ ID NO: 13), miR-6087 (SEQ ID NO: 17) and miR-7641 (SEQ ID NO: 20).

In an embodiment which is not covered by the subject-matter of the claims, one of the nucleic acid(s) capable of specifically binding to miR-4488 or miR-4492 can be replaced with fragments or variants, or modified nucleic acid(s) with additional chemical moiety/moieties capable of specifically binding to at least one miRNA selected from the group consisting of miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30) and miR-1302 (SEQ ID NO: 31).

In an embodiment which is not covered by the subject-matter of the claims, one of the nucleic acid(s) capable of specifically binding to miR-4488 or miR-4492 can be replaced with fragments or variants, or modified nucleic acid(s) with additional chemical moiety/moieties capable of specifically binding to at least one miRNA selected from the group consisting of miR-663a (SEQ ID NO: 4), miR-3184-5p (SEQ ID NO: 5), miR-548ab (SEQ ID NO: 7), miR-3960 (SEQ ID NO: 8), miR-7704 (SEQ ID NO: 9), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-492 (SEQ ID NO: 28), miR-3195 (SEQ ID NO: 32), miR-3196 (SEQ ID NO: 33) and miR-3665 (SEQ ID NO: 34).

An exemplary method for the diagnosis and/or follow-up of cancer in a patient, which is not covered by the subject-matter of the claims, may comprise the steps of:
- evaluating the levels of miR-4488 and/or miR-4492 in a blood sample, a body fluid, or a tissue sample from a patient by using nucleic acid(s) and combinations thereof capable of specifically binding to miR-4488 and/or miR-4492,
- comparing the abundance of miR-4488 and/or miR-4492 with a control sample, and
- determining the presence of cancer or cancer progression depending on alteration in miR-4488 and/or miR-4492 levels as compared to the control.

Said cancer may be selected from the group consisting of colorectal cancer, liver cancer, pancreatic cancer and stomach cancer. Most preferably, it can be colorectal cancer.

The differential expression analysis of miR-4488 and/or miR-4492 combination may be performed in a sample obtained from a patient who has or is suspected of having one of the types of the cancers listed above.

Accordingly, the statistically high/low abundance of miR-4488 and/or miR-4492 in a sample relative to a reference or control sample indicates a higher likelihood of colorectal cancer, hepatocellular carcinoma, pancreatic cancer or stomach cancer. Said sample can be a blood sample, a body fluid, or a tissue sample from a patient Said reference or control sample is a blood sample or a body fluid from a healthy individual, or a healthy tissue sample from a patient.

The expression levels of miR-4488 and/or miR-4492 are considered statistically high/low relative to a reference or control sample when the fold change is higher than 1.5.

A more preferable exemplary method for the diagnosis and/or follow-up of cancer in a patient, which is not covered by the subject-matter of the claims, may comprise the steps of:
- evaluating the levels of miR-4488 and miR-4492 in a blood sample, a body fluid, or a tissue sample from a patient by using nucleic acid(s) and combinations thereof capable of specifically binding to miR-4488 and miR-4492,
- comparing the abundance of miR-4488 and miR-4492 with a control sample, and
- determining the presence of cancer or cancer progression depending on alteration in miR-4488 and miR-4492 levels as compared to the control.

Said cancer may be selected from the group consisting of colorectal cancer, liver cancer, pancreatic cancer and stomach cancer. Most preferably, it can be colorectal cancer.

The differential expression analysis of miR-4488 and miR-4492 combination may be performed in a sample obtained from a patient who has or is suspected of having one of the types of the cancers listed above.

Accordingly, the statistically high/low abundance of miR-4488 and miR-4492 in a sample relative to a reference or control sample indicates a higher likelihood of colorectal cancer, hepatocellular carcinoma, pancreatic cancer or stomach cancer. Said sample can be a blood sample, a body fluid, or a tissue sample from a patient. Said reference or control sample is a blood sample or a body fluid from a healthy individual, or a healthy tissue sample from a patient.

The expression levels of miR-4488 and miR-4492 are considered statistically high/low relative to a reference or control sample when the fold change is higher than 1.5.

In an embodiment which is not covered by the subject-matter of the claims, miRNA molecules of the methods exemplified above can be further selected from the group consisting of miR-4532, miR-3656, miR-6087, and miR-7641.

In another embodiment which is not covered by the subject-matter of the claims, miRNA molecules of the methods exemplified above can be further selected from the group consisting of miR-1267, miR-6801-5p, miR-626, miR-4262, miR-7110-3p, miR-8065, miR-5009-5p, miR-506-3p, miR-33b-5p, miR-3184-3p, and miR-1302.

In a further embodiment which is not covered by the subject-matter of the claims, miRNA molecules of the methods exemplified above can be further selected from the group consisting of miR-663a, miR-3184-5p, miR-548ab, miR-3960, miR-7704, miR-4516, miR-3182, miR-4497, miR-4521, miR-33b-3p, miR-4508, miR-4449, miR-92a-1-5p, miR-492, miR-3195, miR-3196 and miR-3665.

In an embodiment which is not covered by the subject-matter of the claims, the differential expression analysis of miR-4488 and/or miR-4492 along with a further miRNA or combinations thereof selected from the group consisting of miR-1267, miR-663a, miR-3184-5p, miR-6801-5p, miR-548ab, miR-3960, miR-7704, miR-4532, miR-4516, miR-626, miR-3656, miR-3182, miR-4262, miR-4497, miR-6087, miR-7110-3p, miR-4521, miR-7641, miR-8065, miR-5009-5p, miR-33b-3p, miR-4508, miR-4449, miR-92a-1-5p, miR-506-3p, miR-492, miR-33b-5p, miR-3184-3p, miR-1302, miR-3195, miR-3196, and miR-3665 is performed in a sample obtained from a patient The patient has or is suspected of having one of the types of the cancers selected from the group consisting of colorectal cancer, hepatocellular carcinoma, pancreatic cancer and stomach cancer. Said sample can be a blood sample, a body fluid, or a tissue sample from a patient

According to this embodiment which is not covered by the subject-matter of the claims, statistically high/low abundance of miR-4488 and/or miR-4492 along with a further miRNA or combinations thereof selected from the group consisting of miR-1267, miR-663a, miR-3184-5p, miR-6801-5p, miR-548ab, miR-3960, miR-7704, miR-4532, miR-4516, miR-626, miR-3656, miR-3182, miR-4262, miR-4497, miR-6087, miR-7110-3p, miR-4521, miR-7641, miR-8065, miR-5009-5p, miR-33b-3p, miR-4508, miR-4449, miR-92a-1-5p, miR-506-3p, miR-492, miR-33b-5p, miR-3184-3p, miR-1302, miR-3195, miR-3196, and miR-3665 in a sample relative to a reference or control sample indicates a higher likelihood of colorectal cancer, hepatocellular carcinoma, pancreatic cancer or stomach cancer. Said reference or control sample is a blood sample or a body fluid from a healthy individual, or a healthy tissue sample from a patient.

Herein, the expression levels of miR-4488 and/or miR-4492 along with at least one miRNA selected from the group consisting of miR-1267, miR-663a, miR-3184-5p, miR-6801-5p, miR-548ab, miR-3960, miR-7704, miR-4532, miR-4516, miR-626, miR-3656, miR-3182, miR-4262, miR-4497, miR-6087, miR-7110-3p, miR-4521, miR-7641, miR-8065, miR-5009-5p, miR-33b-3p, miR-4508, miR-4449, miR-92a-1-5p, miR-506-3p, miR-492, miR-33b-5p, miR-3184-3p, miR-1302, miR-3195, miR-3196 and miR-3665 are considered statistically high/low relative to a reference or control sample when the fold change is higher than 1.5.

In another embodiment which is not covered by the subject-matter of the claims, one of the nucleic acid(s) capable of specifically binding to miR-4488 or miR-4492 can be replaced with fragments or variants, or modified nucleic acid(s) with additional chemical moiety/moieties capable of specifically binding to at least one miRNA selected from the group consisting of miR-1267, miR-663a, miR-3184-5p, miR-6801-5p, miR-548ab, miR-3960, miR-7704, miR-4532, miR-4516, miR-626, miR-3656, miR-3182, miR-4262, miR-4497, miR-6087, miR-7110-3p, miR-4521, miR-7641, miR-8065, miR-5009-5p, miR-33b-3p, miR-4508, miR-4449, miR-92a-1-5p, miR-506-3p, miR-492, miR-33b-5p, miR-3184-3p, miR-1302, miR-3195, miR-3196 and miR-3665.

In another embodiment which is not covered by the subject-matter of the claims, , nucleic acid(s) and combinations thereof of the present invention comprise nucleic acid(s) capable of specifically binding to miR-4488 (SEQ ID NO: 1) and/or miR-4492 (SEQ ID NO: 2) along with at least one further miRNA selected from the group consisting of miR-1267, miR-663a, miR-3184-5p, miR-6801-5p, miR-548ab, miR-3960, miR-7704, miR-4532, miR-4516, miR-626, miR-3656, miR-3182, miR-4262, miR-4497, miR-6087, miR-7110-3p, miR-4521, miR-7641, miR-8065, miR-5009-5p, miR-33b-3p, miR-4508, miR-4449, miR-92a-1-5p, miR-506-3p, miR-492, miR-33b-5p, miR-3184-3p, miR-1302, miR-3195, miR-3196, and miR-3665. Said miRNA molecules have sequences as provided in SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, and SEQ ID NO: 34, respectively.

Also contemplated but not claimed, is a kit for the diagnosis of cancer consisting nucleic acid(s) and combinations thereof capable of specifically binding to miR-4488 (SEQ ID NO: 1) and/or miR-4492 (SEQ ID NO: 2). More preferably, such embodiment which is not covered by the subject-matter of the claims may comprise a kit for the diagnosis of cancer consisting nucleic acid(s) and combinations thereof capable of specifically binding to miR-4488 (SEQ ID NO: 1) and miR-4492 (SEQ ID NO: 2).

In an aspect, a method for the diagnosis and/or follow-up of colorectal cancer in a patient is provided. The method of the inventioncomprises the steps of:
- evaluating the level of miR-4488 in a blood sample, a body fluid or a tissue sample from a patient by using nucleic acid(s) and combinations thereof capable of specifically binding to miR-4488,
- comparing the abundance of miR-4488 with a control sample, and
- determining the presence of colorectal cancer or colorectal cancer progression depending on alteration in miR-4488 level as compared to the control.

Herein, the method steps may comprise further miRNA or combinations thereof selected from the group consisting of miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) and miR-3665 (SEQ ID NO: 34).

In certain embodiments, each nucleic acid according to the present invention comprise a sequence that is at least 70% complementary to a contiguous 5' to 3' sequence of a respective pri-miRNA, pre-miRNA, mature miRNA, miRNA seed sequence, dsmiRNA and fragments or variants, or modified nucleotide(s) with additional chemical moiety/moieties.

Herein, miR-4488 is hsa-miR-4488, miR-4492 is hsa-miR-4492, miR-1267 is hsa-miR-1267, miR-663a is hsa-miR-663a, miR-3184-5p is hsa-miR-3184-5p, miR-6801-5p is hsa-miR-6801-5p, miR-548ab is hsa-miR-548ab, miR-3960 is hsa-miR-3960, miR-7704 is hsa-miR-7704, miR-4532 is hsa-miR-4532, miR-4516 is hsa-miR-4516, miR-626 is hsa-miR-626, miR-3656 is hsa-miR-3656, miR-3182 is hsa-miR-3182, miR-4262 is hsa-miR-4262, miR-4497 is hsa-miR-4497, miR-6087 is hsa-miR-6087, miR-7110-3p is hsa-miR-7110-3p, miR-4521 is hsa-miR-4521, miR-7641 is hsa-miR-7641, miR-8065 is hsa-miR-8065, miR-5009-5p is hsa-miR-5009-5p, miR-33b-3p is hsa-miR-33b-3p, miR-4508 is hsa-miR-4508, miR-4449 is hsa-miR-4449, miR-92a-1-5p is hsa-miR-92a-1-5p, miR-506-3p is hsa-miR-506-3p, miR-492 is hsa-miR-492, miR-33b-5p is hsa-miR-33b-5p, miR-3184-3p is hsa-miR-3184-3p, miR-1302 is hsa-miR-1302, miR-3195 is hsa-miR-3195, miR-3196 is hsa-miR-3196, and miR-3665 is hsa-miR-3665.

### A) Starvation Process of Cancer Cells

### Cell Culture

MCF-7 breast cancer cells were cultured in Dulbecco's modified Eagle's medium (DMEM, Biological Industries, BI01-050-1A) supplemented with 10% (v/v) fetal bovine serum (FBS, PAN, P30-3302), antibiotics (penicillin/streptomycin, Biological Industries, BI03-031-1B) and L-Glutamine (Biological Industries, BI03-020-1B) in a %5 CO₂ humidified incubator at 37°C. Cells were starved in Earle's Balanced Salt solution (EBSS; Biological Industries, BI02-010-1A) for 4 hours.

### RNA Isolation

Total RNA for miRNA sequencing was extracted using miRVana miRNA Isolation Kit (Thermo, AM1560) according to the manufacturer's instructions. Total RNA for qRT-PCR analysis was extracted using TRIzol reagent (Sigma-Aldrich, T9424) according to the manufacturer's instructions. cDNA was reverse transcribed from DNase-treated total RNA using M-MuLV reverse transcriptase (Fermentas, EP0351) and random hexamers (Invitrogen, 48190-011). For real-time RT-PCR quantification of mRNA levels, SYBR Green Quantitative RT-PCR kit (Roche, 04-913-914-001) and LightCycler 480 (Roche) were used. To activate the SYBR Green, an initial cycle of 95°C, 10 min was performed. PCR reactions were as follows: 95°C for 15 sec and 60°C for 1 min. (40 cycles). Then a thermal denaturation protocol was used to generate the dissociation curves for the verification of amplification specificity (a single cycle of 95°C for 60 sec, 55°C for 60 sec and 80 cycles of 55°C for 10 sec). Changes in mRNA levels were quantified using the 2-^{ΔΔCT} method using U6 mRNA as control. The primers used during RT-PCR are given in Table 1.

**Table 1**

| | |
|---|---|
| MIR4488 stem-loop primer | 5'-GTCGTATCCAGTGCAGGGTCCGAGGTATTCGCACTGGATACGACCGCCGG-3' |
| MIR4488 forward primer | 5'-TTGTTTAGGGGGCGGGCT-3' |
| MIR4492 stem-loop primer | 5'-GTCGTATCCAGTGCAGGGTCCGAGGTATTCGCACTGGATACGACGGCGCG-3' |
| MIR4492 forward primer | 5'-TTTTTTGGGGGCTGGGCG-3' |
| Universal reverse primer | 5'-GTGCAGGGTCCGAGGT-3' |

### miRNA/Small RNA-Sequencing

MiRNA expression analysis was conducted using the NextSeq 500 (Illumina) (ABM, Canada). Total RNA samples were checked for quality using the Agilent 2100 Bioanalyzer platform (Agilent Technologies). All samples passed internal quality control with RNA integrity number (RIN) over eight MiRNA libraries were prepared with the NEBNext Small RNA kit. Project was completed with three sequencing runs. Bcl files were converted to FASTQ data immediately after the run. Over ten million miRNA paired-end reads for all samples.

### miRNA Expression Profiling in Starvation-induced Autophagy

To identify the changes of the miRNA expression pattern during starvation-induced autophagy, two groups of samples were selected: MCF7 cells under normal conditions, and MCF7 cells with starvation treatment for 4 hours. When compared with the control sample, only the miRNAs with fold change ≥2.0 (upregulation) or ≤0.5 (downregulation) and p<0.01 were considered significant

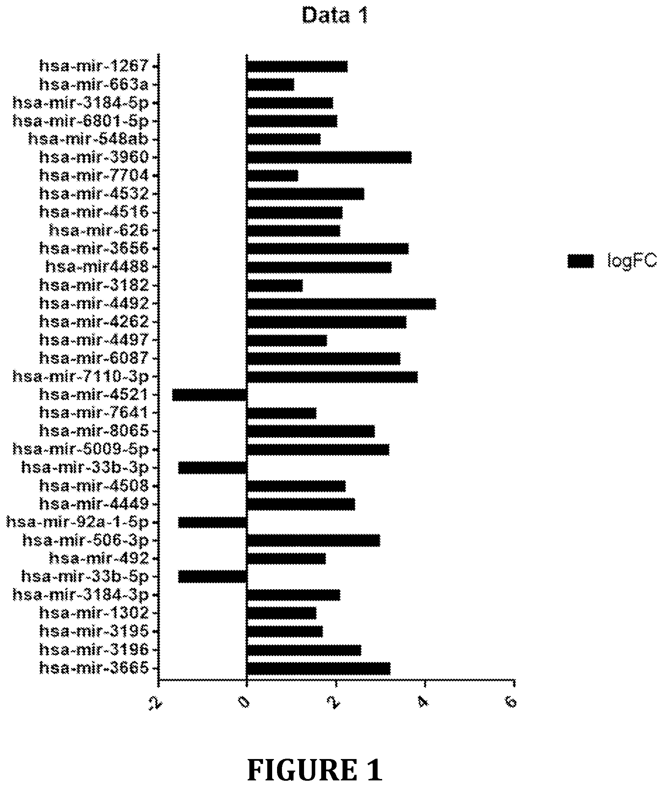

MiRNA expression levels were analyzed for control and starvation-treated samples using microRNA Sequencing. It was shown that 30 miRNAs were significantly upregulated and 4 miRNAs were significantly downregulated (p<0.01). Differentially expressed miRNAs are given in Figure 1. MIR4492 was identified as the miRNA which displayed the highest fold change in an upregulated pattern (log₂ fold change = 4.24), while miR-500-3p displayed the highest fold change in a downregulated pattern in MCF7 cells under starvation treatment (log₂ fold change=-1.70).

### qRT-PCR Validation on Selected miRNA Responses to the Starvation Treatment

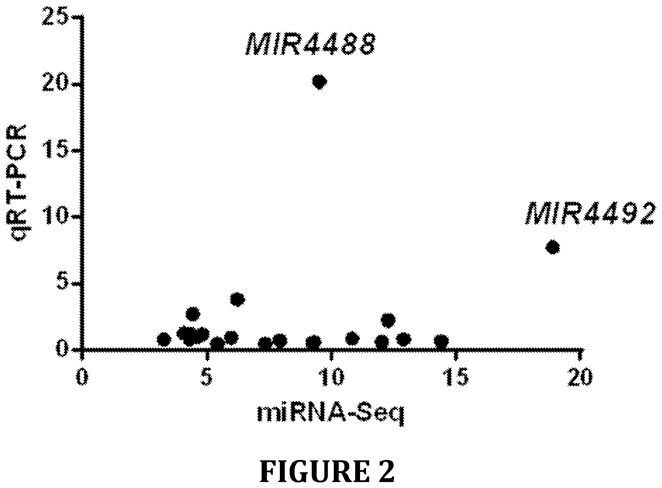

In order to confirm the biological reference from RNA-Seq data, a group of miRNAs were selected for validation by qRT-PCR, based on their fold change (log₂ fold change>2.00). A total of 21 upregulated miRNAs were examined by qRT-PCR in MCF-7 cells with or without starvation treatment (Figure 2). Since the calculation methods are different in qRT-PCR assays and microarray analysis, the values of fold change used in a microarray cannot be directly compared with the fold changes obtained from qRT-PCR assays which are calculated using the 2^{-ΔΔCt} method. Hence, the variation between the miRNA expression from the microarray and qRT-PCR analysis may exist (Draghici et al., 2006). However, comparisons can still be made according to the general trend of up- or downregulation of the miRNA levels via linear analysis.

### B) MiR-4488 and MiR-4492 Expression Analyses

### Patients and Healthy Control Subjects

A total of 48 colorectal cancer patients were enrolled in this study at the Cukurova University Balcal 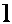 Hospital, Adana. All patients were clinically and pathologically diagnosed with colorectal cancer. Tissue samples were drop frozen in liquid nitrogen shortly after admission. Blood samples (3 mL) were obtained from the elbow vein from patients and healthy control subjects without anticoagulant After centrifugation at 3,500 g for 7 minutes, the supernatant was retained in cryopreservation tubes and stored at -80°C until use. RNA isolation were performed from frozen tissue powders according to the protocols above.

Statistical analyses were performed using Student's two-tailed t-test Data were represented as means of ±SD of n independent experiments (biological replicates). Values of p<0.05 were considered as significant.

### MiR-4488 and MiR-4492 Expression in Tumor Samples in Comparison with Non-tumorous Tissues

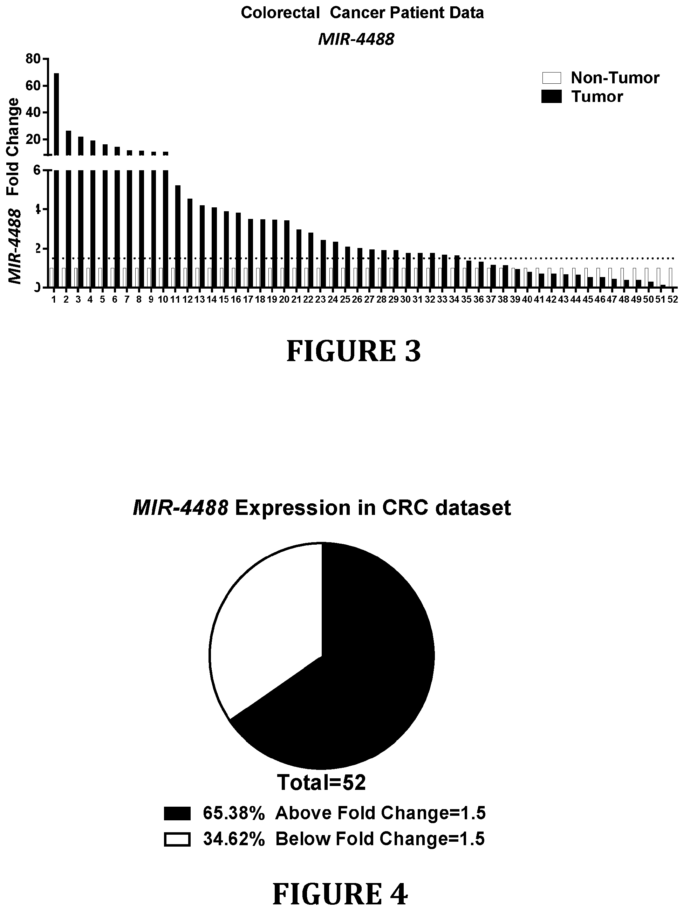

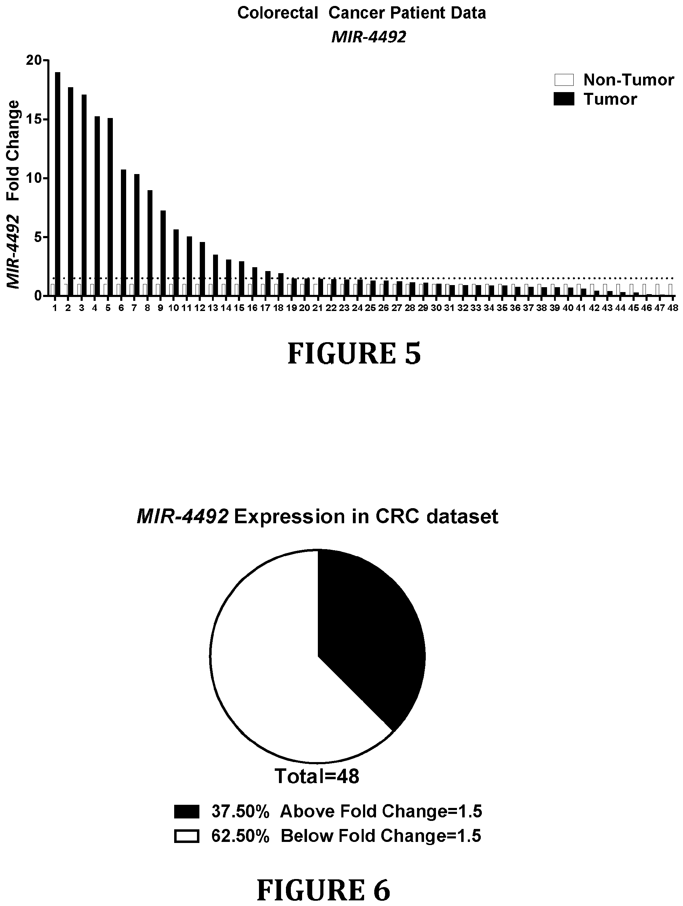

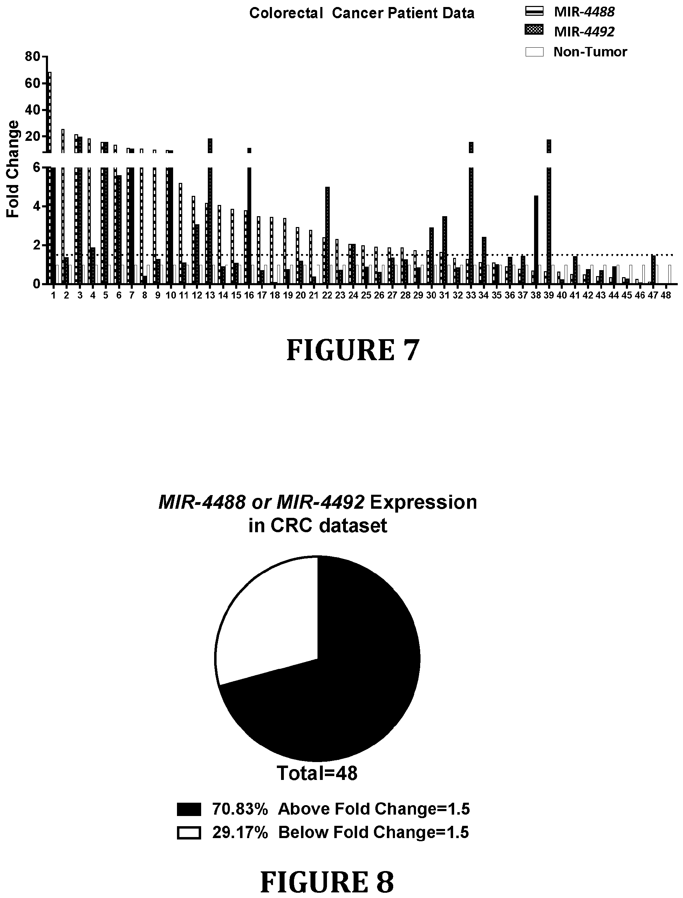

Expression of miR-4488 and miR-4492 in colon cancer tumor tissue samples from patients was analyzed. miR-4488 was upregulated in 65,38% (34/52 samples) (Figures 3 and 4) and miR-4492 in 37,50% (18/48 samples) (Figures 5 and 6) compared to non-tumorous control colon samples from same patients. Moreover, it is observed that upregulation of miR-4488 or miR-4492 showed an overlapping but not identical patient distribution (Figure 7). miR-4488 or miR-4492 were found to be upregulated in 70,83% (34/48 samples) (Figure 8). Therefore, combined detection of both miRNAs in cancer patients might be used as an alternative and highly accurate cancer diagnosis method.

### MIR-4488 and MIR-4492 Expression in Blood Samples

Since diagnostic tests generally performed in patient blood, a series of blood samples from recently diagnosed and pre-op (before cancer operation) CRC patients were obtained and the levels of miR-4488 and miR-4492 in these samples were determined.

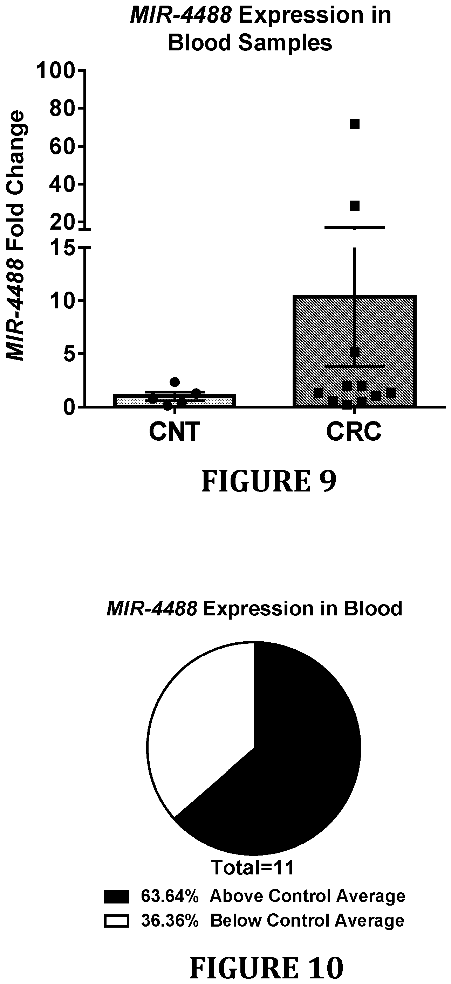

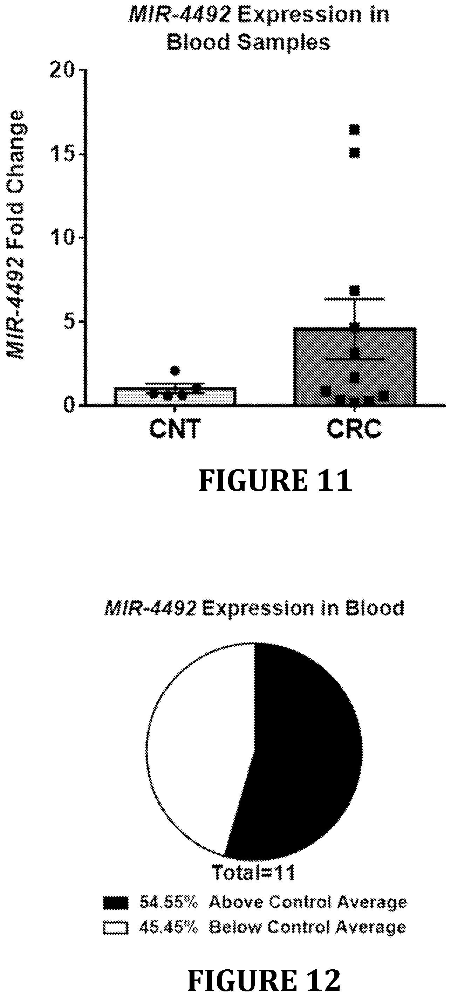

Results of qRT-PCR analysis in blood samples from 5 healthy volunteers and 11 pre-op colon cancer patients showed that expression of both miR-4488 (Figures 9 and 10) and miR-4492 (Figures 11 and 12) were significantly increased in colorectal cancer patients with respect to the control group.

### C) Comparison of MiR-4488 and MiR-4492 to the Classical CRC Markers CEA and CA19-9

Carcinoembryonic antigen (CEA) and carbohydrate antigen 19-9 (CA19-9) are the most widely used biomarkers in the clinic as CRC markers (Berretta et al., 2016). Therefore, in order to evaluate specificity and sensitivity of miRNAs as cancer markers, expression results of miR-4488 and miR-4492 from tumor samples of patients were compared with blood test results of CEA and CA19-9 biomarkers. The results are listed in Table 2 (The values above the threshold are shown in bold). miR-4488 positivity was observed in 26 of 40 patients (65%) whereas miR-4492 was positive in 13 of 38 samples that were analyzed (34,2%). Importantly, positivity for miR-4488 or miR-4492 was observed in 30 out of 40 patients, and reached a detection level of 75%. On the other hand, in the same patient series, CEA or CA19-9 positivity was 37,5% (15/40 patients) and 7,5% (3/40 patients), respectively. Percentage of patients who were positive for either marker was 37,5% (15/40 patients).

These comparisons revealed that specificity and sensitivity of miR-4488, miR-4492 and miR-4488 and miR-4492 combination could diagnose CRC in 75% of patients, outperforming the classical diagnostic biomarkers CEA and CA19-9 (37,5%).

**Table 2**

| **MIR-4488** | **MIR-4492** | **CEA** | **Ca 19-9** |
|---|---|---|---|
| **Fold Change (1,5)** | **Fold Change (1,5)** | **Threshold (5 ng/mL)** | **Threshold (37 U/ml)** |
| 0,758615868 | 1,402109 | 2,29 | 17,2 |
| **1,886662883** | 1,310465 | 1,34 | 10,6 |
| **1,586313121** | - | 1,39 | 17 |
| 0,482460868 | 0,715311 | 1,51 | 14,4 |
| **18,18736626** | **1,832022** | 2,14 | 1,15 |
| 0,637847024 | **16,99861** | 4,27 | 12,14 |
| **1,995010535** | 0,85325 | **33,64** | 14,3 |
| **1,903370759** | 0,558831 | 3,36 | 9,7 |
| 0,49248911 | 1,389801 | 4,04 | 12,5 |
| 0,615743992 | 0,199194 | 1,55 | 2,2 |
| **9,639966111** | **8,904586** | 2,62 | 6,3 |
| 0,397394788 | 0,662688 | **114,1** | **240,2** |
| **1,628831574** | **3,436151** | 4,24 | 15,3 |
| **4,159449203** | **17,6088** | 3,12 | 18,1 |
| **1,712278258** | **2,872196** | **5,46** | 21,7 |
| **2,386730085** | **4,961552** | 4,87 | 9,2 |
| 0,672498096 | - | **5,15** | 8 |
| **2,30402378** | 0,672498 | 1,18 | 9,2 |
| **4,050872388** | 0,86843 | 3,41 | 12,9 |
| **3,376090544** | 0,702808 | **11,54** | 0,5 |
| 0,668556836 | **4,489741** | 4,84 | 4,6 |
| 0,894330875 | 1,34955 | 0,89 | 0,6 |
| **2,931896833** | 1,154893 | **55,99** | 32,8 |
| **3,774990487** | **10,62174** | 1,21 | 25,1 |
| **10,6843522** | 0,366002 | **13,73** | 7,4 |
| **1,722372438** | 0,809286 | **31,7** | **224.0,4** |
| 1,11487304 | **2,379726** | 1,04 | 44 |
| 0,107143167 | 1,414525 | **100,4** | 0,5 |
| 1,333777932 | 0,809286 | **15,95** | 0,5 |
| **25,50066483** | 1,329864 | 4,41 | 0,5 |
| **5,185170175** | 1,066793 | **26,64** | 19,2 |
| **3,853455954** | 1,051231 | **7,57** | 5,7 |
| **3,466580968** | 0,658804 | 3,54 | 24,5 |
| **3,426067366** | 0,050491 | 1,62 | 25 |
| 1,098610134 | 0,968189 | 2,61 | 18,7 |
| 1,27625686 | **15,02472** | **5,13** | 13,1 |
| **13,51628055** | **5,547782** | **5,21** | 27 |
| **4,516208415** | **3,019342** | 1,57 | 9,2 |
| **3,446264634** | - | 3,35 | 9,5 |
| **1,732526126** | - | **8,72** | 14,8 |

### D) MiR-4488 and MiR-4492 Expression in Tumor Samples of Patients with Hepatocellular Carcinoma, Pancreatic Cancer or Stomach Cancer

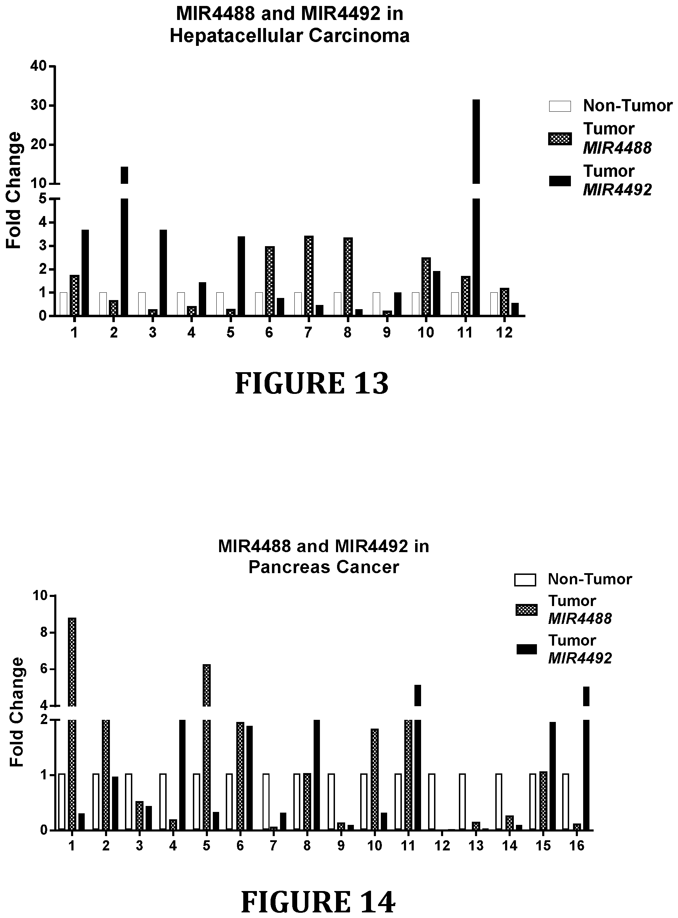

Expression of miR-4488 and miR-4492 in hepatocellular carcinoma tumor tissue samples from patients was analyzed. Determined levels were compared with non-tumor samples. Expression levels of either miR-4488 or miR-4492 is upregulated (>1,5 fold) in 75% of HCC samples (9/12 patients). The results are given in Figure 13.

Expression of miR-4488 and miR-4492 in pancreatic cancer tumor tissue samples from patients was analyzed. Determined levels were compared with non-tumor samples. Expression levels of either miR-4488 or miR-4492 is upregulated (>1,5 fold) in 62,5% of pancreatic cancer samples (10/16 patients). The results are given in Figure 14.

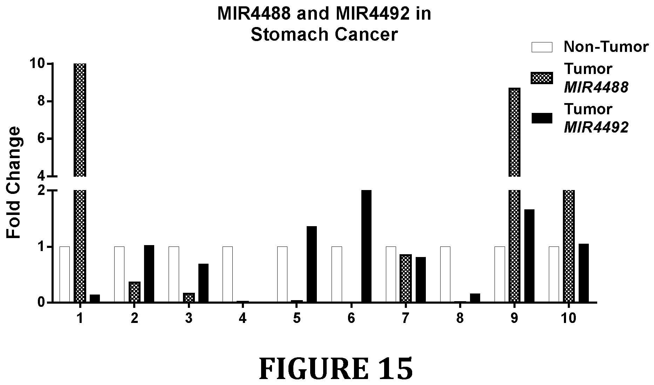

Expression of miR-4488 and miR-4492 in stomach cancer tumor tissue samples from patients was analyzed. Determined levels were compared with non-tumor samples. The results are given in Figure 15.

The sequence numbers of the miRNA molecules of the present invention and their respective miRNA database accession numbers are provided in Table 3.

**Table 3**

| **SEQ ID NO** | **miRNA Name** | **Accession Number** |
|---|---|---|
| SEQ ID NO: 1 | hsa-miR-4488 | MI0016849 |
| SEQ ID NO: 2 | hsa-miR-4492 | MI0016854 |
| SEQ ID NO: 3 | hsa-miR-1267 | MI0006404 |
| SEQ ID NO: 4 | hsa-miR-663a | MI0003672 |
| SEQ ID NO: 5 | hsa-miR-3184-5p | MI0014226 |
| SEQ ID NO: 6 | hsa-miR-6801-5p | MI0022646 |
| SEQ ID NO: 7 | hsa-miR-548ab | MI0016752 |
| SEQ ID NO: 8 | hsa-miR-3960 | MI0016964 |
| SEQ ID NO: 9 | hsa-miR-7704 | MI0025240 |
| SEQ ID NO: 10 | hsa-miR-4532 | MI0016899 |
| SEQ ID NO: 11 | hsa-miR-4516 | MI0016882 |
| SEQ ID NO: 12 | hsa-miR-626 | MI0003640 |
| SEQ ID NO: 13 | hsa-miR-3656 | MI0016056 |
| SEQ ID NO: 14 | hsa-miR-3182 | MI0014224 |
| SEQ ID NO: 15 | hsa-miR-4262 | MI0015872 |
| SEQ ID NO: 16 | hsa-miR-4497 | MI0016859 |
| SEQ ID NO: 17 | hsa-miR-6087 | MI0020364 |
| SEQ ID NO: 18 | hsa-miR-7110-3p | MI0022961 |
| SEQ ID NO: 19 | hsa-miR-4521 | MI0016887 |
| SEQ ID NO: 20 | hsa-miR-7641 | MI0024975 |
| SEQ ID NO: 21 | hsa-miR-8065 | MI0025901 |
| SEQ ID NO: 22 | hsa-miR-5009-5p | MI0017877 |
| SEQ ID NO: 23 | hsa-miR-33b-3p | MI0003646 |
| SEQ ID NO: 24 | hsa-miR-4508 | MI0016872 |
| SEQ ID NO: 25 | hsa-miR-4449 | MI0016792 |
| SEQ ID NO: 26 | hsa-miR-92a-1-5p | MI0000093 |
| SEQ ID NO: 27 | hsa-miR-506-3p | MI0003193 |
| SEQ ID NO: 28 | hsa-miR-492 | MI0003131 |
| SEQ ID NO: 29 | hsa-miR-33b-5p | MI0003646 |
| SEQ ID NO: 30 | hsa-miR-3184-3p | MI0014226 |
| SEQ ID NO: 31 | hsa-miR-1302 | MI0006362 |
| SEQ ID NO: 32 | hsa-miR-3195 | MI0014240 |
| SEQ ID NO: 33 | hsa-miR-3196 | MI0014241 |
| SEQ ID NO: 34 | hsa-miR-3665 | MI0016066 |

## Claims

1. A method for the diagnosis and/or follow-up of colorectal cancer in a patient comprising the steps of:
- evaluating the level of miR-4488 in a blood sample, a body fluid or a tissue sample from a patient by using nucleic acid(s) and combinations thereof capable of specifically binding to miR-4488,
- comparing the abundance of miR-4488 with a control sample, and
- determining the presence of colorectal cancer or colorectal cancer progression depending on alteration in miR-4488 level as compared to the control.

2. A method according to claim 1 wherein the step of evaluating the level of miR-4488 comprises evaluating the levels of a further miRNA or combinations thereof selected from the group consisting of miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) and miR-3665 (SEQ ID NO: 34).

3. A method according to claim 1 wherein the step of comparing the abundance of miR-4488 with a control sample comprises comparing the abundancies of a further miRNA or combinations thereof selected from the group consisting of miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) and miR-3665 (SEQ ID NO: 34).

4. A method according to claim 1 wherein the step of determining the presence of colorectal cancer or colorectal cancer progression comprises determining the presence of colorectal cancer or colorectal cancer progression depending on alteration in the levels of a further miRNA or combinations thereof selected from the group consisting of miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) and miR-3665 (SEQ ID NO: 34) compared to the control.

5. A method according to claim 1 wherein the sample from the patient comprises a blood sample, a body fluid or a tissue sample.

## Patentansprüche

1. Verfahren zur Diagnose und/oder Nachsorge von Darmkrebs bei einem Patienten, umfassend die Schritte:
- Bestimmen des Gehalts an miR-4488 in einer Blutprobe, einer Körperflüssigkeit oder einer Gewebeprobe eines Patienten unter Verwendung von Nukleinsäure(n) und Kombinationen davon, die spezifisch an miR-4488 binden können,
- Vergleichen der Häufigkeit von miR-4488 mit einer Kontrollprobe und
- Bestimmen des Vorliegens von Darmkrebs oder des Fortschreitens von Darmkrebs in Abhängigkeit von einer Veränderung des miR-4488-Gehalts im Vergleich zur Kontrolle.

2. Verfahren nach Anspruch 1, wobei der Schritt des Bestimmens des Gehalts von miR-4488 das Bestimmen der Gehalte einer weiteren miRNA oder Kombinationen davon umfasst, ausgewählt aus der Gruppe, bestehend aus miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) und miR-3665 (SEQ ID NO: 34).

3. Verfahren nach Anspruch 1, wobei der Schritt des Vergleichens der Häufigkeit von miR-4488 mit einer Kontrollprobe das Vergleichen der Häufigkeiten einer weiteren miRNA oder Kombinationen davon umfasst, ausgewählt aus der Gruppe, bestehend aus miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) und miR-3665 (SEQ ID NO: 34).

4. Verfahren nach Anspruch 1, wobei der Schritt des Bestimmens des Vorliegens von Darmkrebs oder des Fortschreitens von Darmkrebs das Bestimmen des Vorliegens von Darmkrebs oder des Fortschreitens von Darmkrebs in Abhängigkeit von einer Veränderung der Gehalte einer weiteren miRNA oder Kombinationen davon umfasst, ausgewählt aus der Gruppe, bestehend aus miR-1267 (SEQ ID NO: 3), miR-6801-5p (SEQ ID NO: 6), miR-626 (SEQ ID NO: 12), miR-4262 (SEQ ID NO: 15), miR-7110-3p (SEQ ID NO: 18), miR-8065 (SEQ ID NO: 21), miR-5009-5p (SEQ ID NO: 22), miR-506-3p (SEQ ID NO: 27), miR-33b-5p (SEQ ID NO: 29), miR-3184-3p (SEQ ID NO: 30), miR-1302 (SEQ ID NO: 31), miR-6087 (SEQ ID NO: 17), miR-548ab (SEQ ID NO: 7), miR-3960 (SEQ ID NO: 8), miR-7704 (SEQ ID NO: 9), miR-492 (SEQ ID NO: 28), miR-4532 (SEQ ID NO: 10), miR-3656 (SEQ ID NO: 13), miR-7641 (SEQ ID NO: 20), miR-663a (SEQ ID NO: 4), miR-3184-5p (SEQ ID NO: 5), miR-4516 (SEQ ID NO: 11), miR-3182 (SEQ ID NO: 14), miR-4497 (SEQ ID NO: 16), miR-4521 (SEQ ID NO: 19), miR-33b-3p (SEQ ID NO: 23), miR-4508 (SEQ ID NO: 24), miR-4449 (SEQ ID NO: 25), miR-92a-1-5p (SEQ ID NO: 26), miR-3195 (SEQ ID NO: 32), miR-3196 (SEQ ID NO: 33) und miR-3665 (SEQ ID NO: 34), im Vergleich zur Kontrolle.

5. Verfahren nach Anspruch 1, wobei die Probe vom Patienten eine Blutprobe, eine Körperflüssigkeit oder eine Gewebeprobe umfasst.

## Revendications

1. Méthode pour le diagnostic et/ou le suivi d'un cancer colorectal chez un patient, comprenant les étapes suivantes :
- évaluation du niveau de miR-4488 dans un échantillon de sang, un fluide corporel ou un échantillon de tissu provenant d'un patient, par utilisation d'un ou plusieurs acides nucléiques et de combinaisons de ceux-ci capables de se lier spécifiquement à miR-4488,
- comparaison de l'abondance de miR-4488 avec un échantillon témoin, et
- détermination de la présence d'un cancer colorectal ou de la progression d'un cancer colorectal en fonction de l'altération du niveau de miR-4488 comparé à celui du témoin.

2. Méthode selon la revendication 1, dans laquelle l'étape d'évaluation du niveau de miR-4488 comprend l'évaluation des niveaux d'un autre ARNmi ou de combinaisons de tels ARNmi choisis dans le groupe constitué par miR-1267 (SEQ ID NO : 3), miR-6801-5p (SEQ ID NO : 6), miR-626 (SEQ ID NO : 12), miR-4262 (SEQ ID NO : 15), miR-7110-3p (SEQ ID NO : 18), miR-8065 (SEQ ID NO : 21), miR-5009-5p (SEQ ID NO : 22), miR-506-3p (SEQ ID NO : 27), miR-33b-5p (SEQ ID NO : 29), miR-3184-3p (SEQ ID NO : 30), miR-1302 (SEQ ID NO : 31), miR-6087 (SEQ ID NO : 17), miR-548ab (SEQ ID NO : 7), miR-3960 (SEQ ID NO : 8), miR-7704 (SEQ ID NO : 9), miR-492 (SEQ ID NO : 28), miR-4532 (SEQ ID NO : 10), miR-3656 (SEQ ID NO : 13), miR-7641 (SEQ ID NO : 20), miR-663a (SEQ ID NO : 4), miR-3184-5p (SEQ ID NO : 5), miR-4516 (SEQ ID NO : 11), miR-3182 (SEQ ID NO : 14), miR-4497 (SEQ ID NO : 16), miR-4521 (SEQ ID NO : 19), miR-33b-3p (SEQ ID NO : 23), miR-4508 (SEQ ID NO : 24), miR-4449 (SEQ ID NO : 25), miR-92a-1-5p (SEQ ID NO : 26), miR-3195 (SEQ ID NO : 32), miR-3196 (SEQ ID NO : 33) et miR-3665 (SEQ ID NO: 34).

3. Méthode selon la revendication 1, dans laquelle l'étape de comparaison de l'abondance de miR-4488 avec un échantillon témoin comprend la comparaison des abondances d'un autre ARNmi ou de combinaisons de tels ARNmi choisis dans le groupe constitué par miR-1267 (SEQ ID NO : 3), miR-6801-5p (SEQ ID NO : 6), miR-626 (SEQ ID NO : 12), miR-4262 (SEQ ID NO : 15), miR-7110-3p (SEQ ID NO : 18), miR-8065 (SEQ ID NO : 21), miR-5009-5p (SEQ ID NO : 22), miR-506-3p (SEQ ID NO : 27), miR-33b-5p (SEQ ID NO : 29), miR-3184-3p (SEQ ID NO : 30), miR-1302 (SEQ ID NO : 31), miR-6087 (SEQ ID NO : 17), miR-548ab (SEQ ID NO : 7), miR-3960 (SEQ ID NO : 8), miR-7704 (SEQ ID NO : 9), miR-492 (SEQ ID NO : 28), miR-4532 (SEQ ID NO : 10), miR-3656 (SEQ ID NO : 13), miR-7641 (SEQ ID NO : 20), miR-663a (SEQ ID NO : 4), miR-3184-5p (SEQ ID NO : 5), miR-4516 (SEQ ID NO : 11), miR-3182 (SEQ ID NO : 14), miR-4497 (SEQ ID NO : 16), miR-4521 (SEQ ID NO : 19), miR-33b-3p (SEQ ID NO : 23), miR-4508 (SEQ ID NO : 24), miR-4449 (SEQ ID NO : 25), miR-92a-1-5p (SEQ ID NO : 26), miR-3195 (SEQ ID NO : 32), miR-3196 (SEQ ID NO : 33) et miR-3665 (SEQ ID NO: 34).

4. Méthode selon la revendication 1, dans laquelle l'étape de détermination de la présence d'un cancer colorectal ou de la progression d'un cancer colorectal comprend la détermination de la présence d'un cancer colorectal ou de la progression d'un cancer colorectal en fonction d'altérations des niveaux d'un autre ARNmi ou de combinaisons de tels ARNmi choisis dans le groupe constitué par miR-1267 (SEQ ID NO : 3), miR-6801-5p (SEQ ID NO : 6), miR-626 (SEQ ID NO : 12), miR-4262 (SEQ ID NO : 15), miR-7110-3p (SEQ ID NO : 18), miR-8065 (SEQ ID NO : 21), miR-5009-5p (SEQ ID NO : 22), miR-506-3p (SEQ ID NO : 27), miR-33b-5p (SEQ ID NO : 29), miR-3184-3p (SEQ ID NO : 30), miR-1302 (SEQ ID NO : 31), miR-6087 (SEQ ID NO : 17), miR-548ab (SEQ ID NO : 7), miR-3960 (SEQ ID NO : 8), miR-7704 (SEQ ID NO : 9), miR-492 (SEQ ID NO : 28), miR-4532 (SEQ ID NO : 10), miR-3656 (SEQ ID NO : 13), miR-7641 (SEQ ID NO : 20), miR-663a (SEQ ID NO : 4), miR-3184-5p (SEQ ID NO : 5), miR-4516 (SEQ ID NO : 11), miR-3182 (SEQ ID NO : 14), miR-4497 (SEQ ID NO : 16), miR-4521 (SEQ ID NO : 19), miR-33b-3p (SEQ ID NO : 23), miR-4508 (SEQ ID NO : 24), miR-4449 (SEQ ID NO : 25), miR-92a-1-5p (SEQ ID NO : 26), miR-3195 (SEQ ID NO : 32), miR-3196 (SEQ ID NO : 33) et miR-3665 (SEQ ID NO: 34), comparés à ceux du témoins.

5. Méthode selon la revendication 1, dans laquelle l'échantillon provenant du patient comprend un échantillon de sang, un fluide corporel ou un échantillon de tissu.


### Sequence Listing

<110> Sabanc 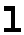 University
<120> Stress-Regulated miRNAs As Novel Cancer Biomarkers
<130> 5
<160> 34
<170> Patent In version 3.5
<210> 1
   <211> 62
   <212> RNA
   <213> Homo sapiens
<400> 1
   gguagggggc gggcuccggc gcugggaccc cacuagggug gcgccuuggc cccgccccgc    60
   cc                                                                  62
<210> 2
   <211> 80
   <212> RNA
   <213> Homo sapiens
<400> 2
   cugcagcgug cuucuccagg ccccgcgcgc ggacagacac acggacaagu cccgccaggg    60
   gcugggcgcg cgccagccgg                                                80
<210> 3
   <211> 78
   <212> RNA
   <213> Homo sapiens
<400> 3
   cucccaaauc uccuguugaa guguaauccc caccuccagc auuggggauu acauuucaac 60
   augagauuug gaugagga 78
<210> 4
   <211> 93
   <212> RNA
   <213> Homo sapiens
<400> 4
   ccuuccggcg ucccaggcgg ggcgccgcgg gaccgcccuc gugucugugg cggugggauc 60
   ccgcggccgu guuuuccugg uggcccggcc aug 93
<210> 5
   <211> 75
   <212> RNA
   <213> Homo sapiens
<400> 5
   aagcaagacu gaggggccuc agaccgagcu uuuggaaaau agaaaagucu cgcucucugc 60
   cccucagccu aacuu 75
<210> 6
   <211> 79
   <212> RNA
   <213> Homo sapiens
<400> 6
   uggccugguc agaggcagca ggaaaugaga guuagccagg agcuuugcau acucaccccu 60
   gccacucacu ggcccccag 79
<210> 7
   <211> 84
   <212> RNA
   <213> Homo sapiens
<400> 7
   auguuggugc aaaaguaauu guggauuuug cuauuacuug uauuuauuug uaaugcaaaa 60
   cccgcaauua guuuugcacc aacc 84
<210> 8
   <211> 91
   <212> RNA
   <213> Homo sapiens
<400> 8
   ggcgccccgg cuccccgcgc ccccgaucgg ggccgccgcu aguaguggcg gcggcggagg 60
   cgggggcagc ggcggcggcg gcggaggcgc c 91
<210> 9
   <211> 59
   <212> RNA
   <213> Homo sapiens
<400> 9
   cggggucggc ggcgacgugc ucagcuuggc acccaaguuc ugccgcuccg acgcccggc 59
<210> 10
   <211> 51
   <212> RNA
   <213> Homo sapiens
<400> 10
   acagaccccg gggagcccgg cggugaagcu ccugguaucc ugggugucug a 51
<210> 11
   <211> 86
   <212> RNA
   <213> Homo sapiens
<400> 11
   agggagaagg gucggggcag ggagggcagg gcaggcucug gggugggggg ucugugaguc 60
   agccacggcu cugcccacgu cucccc 86
<210> 12
   <211> 94
   <212> RNA
   <213> Homo sapiens
<400> 12
   acugauauau uugucuuauu ugagagcuga ggaguauuuu uaugcaaucu gaaugaucuc 60
   agcugucuga aaaugucuuc aauuuuaaag gcuu 94
<210> 13
   <211> 69
   <212> RNA
   <213> Homo sapiens
<400> 13
   cuuucggcca gcgggacggc auccgaggug ggcuaggcuc gggcccgugg cgggugcggg 60
   ggugggagg 69
<210> 14
   <211> 63
   <212> RNA
   <213> Homo sapiens
<400> 14
   gcugcuucug uaguguaguc cgugcauccg cccuucgaug cuuggguugg aucauagagc 60
   agu 63
<210> 15
   <211> 54
   <212> RNA
   <213> Homo sapiens
<400> 15
   gaaagcugca ggugcugaug uuggggggac auucagacua ccugcagcag agcc 54
<210> 16
   <211> 89
   <212> RNA
   <213> Homo sapiens
<400> 16
   accuccggga cggcugggcg ccggcggccg ggagauccgc gcuuccugaa ucccggccgg 60
   cccgcccggc gcccguccgc ccgcggguc 89
<210> 17
   <211> 49
   <212> RNA
   <213> Homo sapiens
<400> 17
   ggugaggcgg gggggcgagc ccugaggggc ucucgcuucu ggcgccaag 49
<210> 18
   <211> 86
   <212> RNA
   <213> Homo sapiens
<400> 18
   ggggcugggg guguggggag agagagugca cagccagcuc agggauuaaa gcucuuucuc 60
   ucucucucuc ucccacuucc cugcag 86
<210> 19
   <211> 60
   <212> RNA
   <213> Homo sapiens
<400> 19
   ucggcuaagg aaguccugug cucaguuuug uagcaucaaa acuaggauuu cucuuguuac 60
<210> 20
   <211> 61
   <212> RNA
   <213> Homo sapiens
<400> 20
   ucucguuuga ucucggaagc uaagcagggu ugggccuggu uaguacuugg augggaaacu 60
   u 61
<210> 21
   <211> 100
   <212> RNA
   <213> Homo sapiens
<400> 21
   caguacauga gagccaucau uuuuguuguu guuguauuuu ucagcaaggc aagggcuuug 60
   augucuugua ggaacaguug aauuuuggcu ucauguaauu 100
<210> 22
   <211> 100
   <212> RNA
   <213> Homo sapiens
<400> 22
   gaccagaagu guuuuggauu uuggacuuuu ucagauuugg ggauauuugc auuauacuua 60
   uccuaaaucu gaaaguccaa aaccugaaau gaccaauaag 100
<210> 23
   <211> 96
   <212> RNA
   <213> Homo sapiens
<400> 23
   gcgggcggcc ccgcggugca uugcuguugc auugcacgug ugugaggcgg gugcagugcc 60
   ucggcagugc agcccggagc cggccccugg caccac 96
<210> 24
   <211> 70
   <212> RNA
   <213> Homo sapiens
<400> 24
   aggacccagc ggggcugggc gcgcggagca gcgcugggug cagcgccugc gccggcagcu 60
   gcaagggccg 70
<210> 25
   <211> 66
   <212> RNA
   <213> Homo sapiens
<400> 25
   agcagcccuc ggcggcccgg ggggcgggcg gcggugcccg ucccggggcu gcgcgaggca 60
   caggcg 66
<210> 26
   <211> 78
   <212> RNA
   <213> Homo sapiens
<400> 26
   cuuucuacac agguugggau cgguugcaau gcuguguuuc uguaugguau ugcacuuguc 60
   ccggccuguu gaguuugg 78
<210> 27
   <211> 124
   <212> RNA
   <213> Homo sapiens
<400> 27
   gccaccacca ucagccauac uauguguagu gccuuauuca ggaagguguu acuuaauaga 60
   uuaauauuug uaaggcaccc uucugaguag aguaaugugc aacauggaca acauuugugg 120
   uggc 124
<210> 28
   <211> 116
   <212> RNA
   <213> Homo sapiens
<400> 28
   caacuacagc cacuacuaca ggaccaucga ggaccugcgg gacaagauuc uuggugccac 60
   cauugagaac gccaggauug uccugcagau caacaaugcu caacuggcug cagaug 116
<210> 29
   <211> 96
   <212> RNA
   <213> Homo sapiens
<400> 29
   gcgggcggcc ccgcggugca uugcuguugc auugcacgug ugugaggcgg gugcagugcc 60
   ucggcagugc agcccggagc cggccccugg caccac 96
<210> 30
   <211> 75
   <212> RNA
   <213> Homo sapiens
<400> 30
   aagcaagacu gaggggccuc agaccgagcu uuuggaaaau agaaaagucu cgcucucugc 60
   cccucagccu aacuu 75
<210> 31
   <211> 143
   <212> RNA
   <213> Homo sapiens
<400> 31
   cagaaagccc aguuaaauuu gaauuucaag uaaacaauga auaauugugu auguaagaau      60
   aucccauaca auauuuggga cauacuuaug cuaaaaauua uuccuugcuu aucugaaauu     120
   caaauguaac uaggauuccu gua                                             143
<210> 32
   <211> 84
   <212> RNA
   <213> Homo sapiens
<400> 32
   ccgcagccgc cgcgccgggc ccggguuggc cgcugacccc cgcggggccc ccggcggccg      60
   gggcgggggc gggggcugcc ccgg                                             84
<210> 33
   <211> 64
   <212> RNA
   <213> Homo sapiens
<400> 33
   gggugggggc ggggcggcag gggccucccc cagugccagg ccccauucug cuucucuccc      60
   agcu                                                                  64
<210> 34
   <211> 105
   <212> RNA
   <213> Homo sapiens
<400> 34
   gcgggcggcg gcggcggcag cagcagcagg ugcggggcgg cggccgcgcu ggccgcucga      60
   cuccgcagcu gcucguucug cuucuccagc uugcgcacca gcucc                    105